# EUROPÄISCHE PATENTSCHRIFT

(11) **EP 2 708 198 B1**
(45) Veröffentlichungstag und Bekanntmachung des Hinweises auf die Patenterteilung: **07.01.2015**
(21) Anmeldenummer: 12184854.3
(22) Anmeldetag: 18.09.2012
(51) Int. Cl.: A61B 17/80, A61B 19/00

(54) **Brustbeinverschluss nach Art einer Sternal Plate mit einem integralen Durchtrennungsstellenbauteil**
Sternum closure in the form of a sternal plate with an integral separation component
Fermeture du sternum selon la méthode d'une plaque sternale avec composant de séparation intégral

(43) Veröffentlichungstag der Anmeldung: 19.03.2014
(73) Patentinhaber: Karl Leibinger Medizintechnik GmbH & Co. KG, 78570 Mühlheim/Donau (DE)
(72) Erfinder: Waizenegger, Axel, 78570 Mühlheim (DE); Koett Thomas, 78600 Kolbingen (DE)
(74) Vertreter: Winter, Brandl, Fürniss, Hübner, Röss, Kaiser, Polte - Partnerschaft mbB

(56) Entgegenhaltungen:
- EP-A1- 1 654 994
- WO-A2-2008/073898
- US-A1- 2002 128 654
- US-A1- 2006 015 103

## Beschreibung

Die Erfindung betrifft einen Brustbeinverschluss zum Verschließen eines Spaltes in einem Brustbein nach dem Oberbegriff des Anspruchs 1.

Solche Brustbeinverschlüsse werden zum Verschließen eines menschlichen Brustbeines, das vorher geteilt wurde oder eine Fraktur aufweist, eingesetzt. Das Teilen von Brustbeinen ist insbesondere bei Herzoperationen notwendig. Allerdings heilt das Brustbein relativ langsam, weswegen eine die Heilung unterstützende Verschlusseinrichtung im Regelfall einzusetzen ist.

Herkömmlicherweise werden Titan-Drähte verwendet, wie sie bspw. aus der DE 20 2010 012 426 U1 bekannt sind.

Kabelbinderartige Sternum-Schließverbindungen sind auch aus der DE 87 03 432 U1 bekannt. Dort wird eine Sternum-Schließvorrichtung offenbart, die ein Kopfteil, ein flexibles Rückgratteil und ein Schwanzteil umfasst, wobei das Schwanzteil und das Rückgratteil im Kopfteil aufnehmbar sind, welche eine Verriegelungseinrichtung enthält, an der das Rückgratteil so angreift, dass dann, wenn das Rückgratteil im Kopfteil aufgenommen ist, es an einer Rückwärtsbewegung gehindert ist, wobei ferner das Rückgratteil einen oberen Rand und einen unteren Rand aufweist, zwischen denen eine Verzahnung liegt, wobei ferner der Schwanzteil eine geschärfte Nadel aufweist, die mit dem Rückgratteil integral ausgebildet ist, um ein Durchstechen des Zwischenrippengewebes zu ermöglichen.

Eine Brustbeinverschlussvorrichtung ist auch aus der US 2007/0038218 A1 bekannt. Dort werden Stangen in Längsrichtung des Brustbeins auf diesen mittels Verschraubungen angebracht, wobei separate Verbindungselemente an den Stangen befestigt werden.

Vorrichtungen zur Fixierung eines Sternums sind auch aus der DE 602 08 880 P2 bekannt. Die dort offenbarte Sternum-Fixationsvorrichtung zur Befestigung von Teilen eines Sternums weist eine erste Platte mit einer oberen Oberfläche und einer für den Kontakt zum Sternum bestimmten Oberfläche auf, wobei eine zweite Platte mit zumindest einem hakenförmigen Bauteil zur Fixation am Sternum und einem ausklinkbaren Bauteil zum Zusammenhalten der ersten und zweiten Platte eingesetzt ist, wobei ferner das ausklinkbare Bauteil bewegbar mit mindestens einer der ersten und zweiten Platten verbunden ist, so dass es zur Separation der zwei Teile des Sternums bewegt werden kann, wobei insbesondere die erste Platte mindestens ein die obere und die für den Kontakt mit dem Sternum bestimmte Oberfläche durchdringendes Loch zur Aufnahme eines Desinfektionsmittels aufweist.

Die bekannten Vorrichtungen haben jedoch zahlreiche Nachteile, die es abzustellen gilt. So schneidet bspw. die Titandrahtvariante sehr stark in das menschliche Gewebe ein, so dass Komplikationen nicht auszuschließen sind.

Die letztgenannte Vorrichtung ist sehr aufwändig und kostenintensiv, was dazu führt, dass solche Vorrichtungen nur ausnahmsweise zum Einsatz kommen.

Auch die mit den zwei Stangen arbeitende Lösung über ein separates Verbindungsteil, ist sehr aufwändig in der Montage und diesbezüglich zu verbessern.

Weiterer Stand der Technik ist aus der US 2002/128654 A1 und der EP 1 654 994 A1 bekannt. Als nächstliegender Stand der Technik wird die US 2002/128654 A1 angesehen, die die Merkmale des Oberbegriffs des Anspruchs 1 offenbart.

### Offenbarung der Erfindung

Ein gattungsgemäßer Brustbeinverschluss wird durch die Merkmale des kennzeichnenden Teils des Anspruchs 1 verbessert. Ein solches Durchtrennungsstellenteil bzw. -bauteil oder Durchtrennungsteil bzw. -bauteil ist von den Befestigungsbereichen entfernbar, wenn eine Durchtrennung des Brustbeinverschlusses vorgenommen wird.

Zur Abgrenzung sei erwähnt: Während eine Sollbruchstelle ein durch konstruktive oder mechanische bzw. physikalische Maßnahmen oder Auslegungen vorgesehenes Konstruktionselement ist, und im Schadens- oder Überlastfall dieses Element gezielt und vorhersagbar versagen wird, um hierdurch einen möglichen Schaden in einem Gesamtsystem klein zu halten oder eine besondere Funktion zu erreichen, soll bei dem verbautem Brustbeinverbinder selbst im über das normale Maß hinausgehenden Belastungsfall kein Ausfall und somit kein Bruch an einer "Sollbruchstelle" stattfinden. Die Verwendung einer Kerbe oder einer Einritzung ist, obwohl möglich, nicht nötig. Eine durch die Kerbwirkung auf das Bauteil schwächende Auswirkung ist vermeidbar.

Vorteilhafterweise wird eine Sollbruchstelle vermieden und vielmehr an einer definierten Stelle, die für ein aktives Durchtrennen, etwa mittels eines Werkzeugs, vorbereitet ist, ein Heraustrennen des Durchtrennungsbauteil möglich. Das Durchtrennungsbauteil wirkt dann wie ein mittig angeordnetes Entnahmeteil / -bauteil. An der definierten Stelle kann das plattenartige Durchtrennungsbauteil vom Rest getrennt werden bzw. ein Element herausgetrennt werden.

Dieses Entnahmeteil hat den größten Mehrwert. Durch dieses Entnehmen des "Mittelteils", d.h. Durchtrennteil oder Durchtrennungsstellenbauteil, wird ein relativ breiter Zugang zum Sternum frei, welcher wenn notwendig im OP wieder getrennt werden kann ohne irgendwelche Schrauben zu entfernen. Das Durchtrennungsstellenteil ist mittig, etwa sternumzentral, bzw. für eine "Midline Sternotomie" angepasst, angeordnet.

Die Montage, d.h. das operative Verbauen des Brustbeinverschlusses wird dann einfach möglich, wobei selbst während einer Notoperation eine Schnellöffnung des Brustbeins möglich ist, um dann auch schnell und komplikationsfrei an die unter dem Brustbein liegenden Organe zu gelangen. Mit anderen Worten wird hier eine Verbindungsplatte für einen Brustbeinverschluss verwendet, wobei die Platte beidseitig Bohrungen aufweist, die mit Schrauben bestückt werden können, um das Brustbein bzw. die Rippen zu fixieren.

Vorteilhafte Ausführungsformen sind in den Unteransprüchen beansprucht und werden nachfolgend näher erläutert.

So ist es von Vorteil, wenn das Durchtrennungsstellenteil einen geschwächten Durchmesserbereich, der auch als Durchtrennungsbereich agiert und entsprechend bezeichnet werden kann, aufweist. Mit anderen Worten weist das Durchtrennungsstellenteil also eine Durchtrennungsstelle auf, ergo eine Stelle, die für ein gewolltes Durchtrennen vorbereitet ist. An einer solchen Stelle ist ein schnelles Trennen des Brustbeinverschlusses selbst in kritischen Situationen, wie einer Notoperation möglich. Keine aufwändigen Prozeduren müssen durchlaufen werden, wie z.B. das Entnehmen von Schrauben. Der geschwächte Durchmesserbereich kann auch dadurch geschaffen werden, dass das Material in diesem Bereich besonders wenig Bruchwiderstand leistet. Ein entsprechendes Einwirken auf die Gefügestruktur ist hierbei von Vorteil. Es können auch mehrere Bereiche entsprechend dieser Definition vorbereitet sein, insbesondere zwei oder vier Stellen.

Wenn die beiden Befestigungsbereiche zumindest, in den aufeinander zu zeigenden Endbereichen in einer gemeinsamen ersten Ebene liegen, so lässt sich das Anbringen an den Rippen bzw. am Brustbein besonders problemfrei bewerkstelligen.

Um ein effizientes Funktionieren zu gewährleisten, ist es von Vorteil, wenn das Durchtrennungsstellenteil in einer zweiten Ebene befindlich ist, welches zur ersten Ebene beabstandet und vorzugsweise parallel verlaufend ist. Ein vorteilhaftes Ausführungsbeispiel ist auch dadurch gekennzeichnet, dass das Durchtrennungsstellenteil auf der brustbeinfernen Seite der ersten Ebene befindlich ist. Während somit eine Unterseite des Befestigungsbereiches bzw. die Unterseite beider Befestigungsbereiche zur Anlage an das Brustbein vorgesehen ist, ist von der Unterseite beabstandet das Durchtrennungsstellenteil konfiguriert, um für einen durchtrennenden Angriff eines Werkzeuges, wie einer Zange vorbereitet zu sein. Dadurch wird ein besonders schnelles Öffnen des Brustbeinverschlusses ermöglicht.

Als besonders vorteilhaft in puncto Belastbarkeit hat es sich herausgestellt, wenn einer der beiden Befestigungsbereiche einen Verlängerungsabschnitt aufweist, der zwischen dem Brustbein und dem Durchtrennungsstellenteil befindlich ist, wobei der Verlängerungsabschnitt vom Durchtrennungsstellenteil beabstandet ist, aber am Brustbein in Anlage bringbar ist und dabei die Dicke des Verlängerungsabschnittes das 0,4- bis 0,7-fache, besonders vorteilhaft das 0,5-fache der Dicke des Durchtrennungsstellenteils aufweist, so stellen sich neben Kostenvorteilen auch Stabilitätsvorteile ein. Besonders vorteilhaft ist es, wenn der Verlängerungsabschnitt eine Dicke (auf das Brustbein zu gemessen) von 0,9 mm und das Durchtrennungsstellenteil eine Dicke von 1,9 mm aufweist. Ist der Abstand zwischen dem Verlängerungsabschnitt und dem Durchtrennungsstellenteil das 0,5-bis 0,9-fache der Dicke des Verlängerungsabschnittes und/oder das 0,2- bis 0,5-fache der Dicke des Durchtrennungsstellenteils, so werden besonders dünne aber belastbare Brustbeinverschlüsse konfigurierbar.

Es ist ferner von Vorteil, wenn im Verlängerungsabschnitt ein Loch für eine Knochenschraube vorhanden ist. Die präzise Positionierung der einzelnen Abschnitte ist dann möglich.

Zweckmäßig ist es auch, wenn in jedem der beiden länglichen, in Flucht zueinander ausgerichteten Befestigungsabschnitte eine Vielzahl gleichmäßig verteilter Löcher vorhanden ist. Die Befestigungsabschnitte können dann entlang einer Rippe bzw. zweier Rippen befestigt werden, wobei dann eine ausreichende Festigkeit bewirkt werden kann. Um die Durchtrennbarkeit während einer Notoperation besonders gut zu gewährleisten, ist es von Vorteil, wenn das Durchtrennungsstellenteil eine maximale Breite aufweist, die größer ist, als die Breite der Befestigungsbereiche. Der Operateur gelangt mit seinem Werkzeug, wie der Zange, als erstes somit an das Durchtrennungsstellenteil und kann dieses problemlos durchtrennen. Wenn das Durchtrennungsstellenteil zumindest einen Hohlraum oder eine Vielzahl an Hohlräume aufweist, so kann der Brustbeinverschluss besonders leicht und materialeffizient ausgestaltet sein. Auch die Durchtrennbarkeit wird nicht eingeschränkt.

Als besonders effizient hat es sich auch herausgestellt, wenn das Durchtrennungsstellenteil zwei Stäbe aufweist, die vorzugsweise die maximale Breite des Durchtrennungsstellenteils festlegen.

Zweckmäßig ist es dabei, wenn ein Stab oder jeder Stab über eine oder zwei Verdünnungsstellen mit einem Befestigungsbereich oder beiden Befestigungsbereichen verbunden ist. Gerade in der oder den Verdünnungsstellen lässt sich dann das Durchtrennen des Brustbeinverschlusses wirkungsvoll vornehmen.

Als besonders belastbar hat es sich herausgestellt, wenn das Durchtrennungsstellenteil eine fachwerkartige Struktur oder eine hantelartige oder doppel-gabelartige Struktur aufweist.

Zweckmäßig ist es auch, wenn die Durchtrennungsstelle und/oder die Verdünnungen in einem außermittigen Bereich des Durchtrennungsstellenteils vorhanden ist/sind.

Ein vorteilhaftes Ausführungsbeispiel ist auch dadurch gekennzeichnet, dass der Brustbeinverschluss aus einer Metalllegierung, wie einer Titanlegierung und/oder Kunststoff aufgebaut ist.

Es ist auch von Vorteil, wenn das Durchtrennungsstellenteil mittig über einer Mittelline des Sternums angeordnet ist, wobei die Durchtrennung des Sternums im Bereich dieser Mittelline vorgenommen wird oder vorhanden ist.

Dabei ist es auch einer schnellen Not-Entnahme des Durchtrennungsstellenteils zuträglich, wenn das Durchtrennungsstellenteil über kraft- und/oder form- und/oder stoffschlüssige Verbindungsarten, bspw. mittels Schrauben und/oder Stiften und/oder Zapfen eine Lösbarkeit ermöglicht wird, weiter vorzugsweise ohne Einsatz eines bspw. schneidenden Werkzeugs.

Auf diese Weise wird somit eine Platte geschaffen, die an mindestens zwei Stellen einen Querschnitt aufweist, der derart verdünnt ist, dass ein leichtes Durchtrennen möglich ist und der Zugang eines "cutters" optimiert ist. Die Stelle, an der die Durchtrennung stattfinden soll, kann ebenfalls nach oben versetzt ausgeführt werden, damit keine Auflage auf dem Knochen stattfindet. Das Durchtrennen der Platte findet nicht mittig, sondern au ßermittig statt. So kann gewährleistet werden, dass ein mittleres Stück entnommen werden kann und erneut eine "midline sternotomy" durchgeführt werden kann. Dies ist bei einer mittigen Durchtrennung nur schwer möglich. Ebenso müssen bei einer Not-Operation keine Schrauben frei präpariert und entfernt werden. Dies bedeutet für den Operateur einen großen Vorteil an Zeiteinsparung und für den Patienten die Chance, dass das Brustbein nach der Durchtrennung wieder mit "Standard-Lösungen" verschlossen werden kann.

Ferner ist es von Vorteil, wenn eine Zusatzplatte und/oder ein Zusatzelement vorgehalten wird und in den Brustbeinverschluss eingesetzt ist, so dass die vom Durchtrennungsstellenteil befreiten Enden des Brustbeinverschlusses kraft- und/oder form- und/oder stoffschlüssig überbrückt sind (wenn der Noteingriff in den Körper des Menschen abgeschlossen ist) und dabei die Enden mittels des Durchtrennungsstellenteils (zum Kraftübertragen vorbereitet) miteinander verbunden sind.

Die Erfindung wird nachfolgend auch mit Hilfe einer Zeichnung näher erläutert, in der unterschiedliche Ausführungsbeispiele dargestellt sind. Es zeigen:
- Fig. 1: eine erste Ausführungsform eines erfindungsgemäßen Brustbeinverschlusses in einer perspektivischen Ansicht,
- Fig. 2: eine Ansicht auf den Brustbeinverschluss aus Fig. 1 in seiner gesamten Erstreckung, in einer zur Fig. 1 ähnlichen Darstellungsart,
- Fig. 3: eine Detailansicht des Bereichs III aus Fig. 2,
- Fig. 4: eine Darstellung des Ausschnittes III aus Fig. 2 in einer zur Fig. 3 gedrehten Darstellung,
- Fig. 5: einen Ausschnitt einer weiteren Variante eines nicht erfindungsgemäßen Brustbeinverschlusses,
- Fig. 6: einen Ausschnitt einer weiteren Variante eines nicht erfindungsgemäßen Brustbeinverschlusses,
- Fig. 7: einen Ausschnitt einer weiteren Variante eines nicht erfindungsgemäßen Brustbeinverschlusses, und
- Fig. 8: einen Ausschnitt einer fünften Variante eines Brustbeinverschlusses.

Die Figuren sind lediglich schematischer Natur und dienen nur dem Verständnis der Erfindung. Die gleichen Elemente sind mit denselben Bezugszeichen versehen.

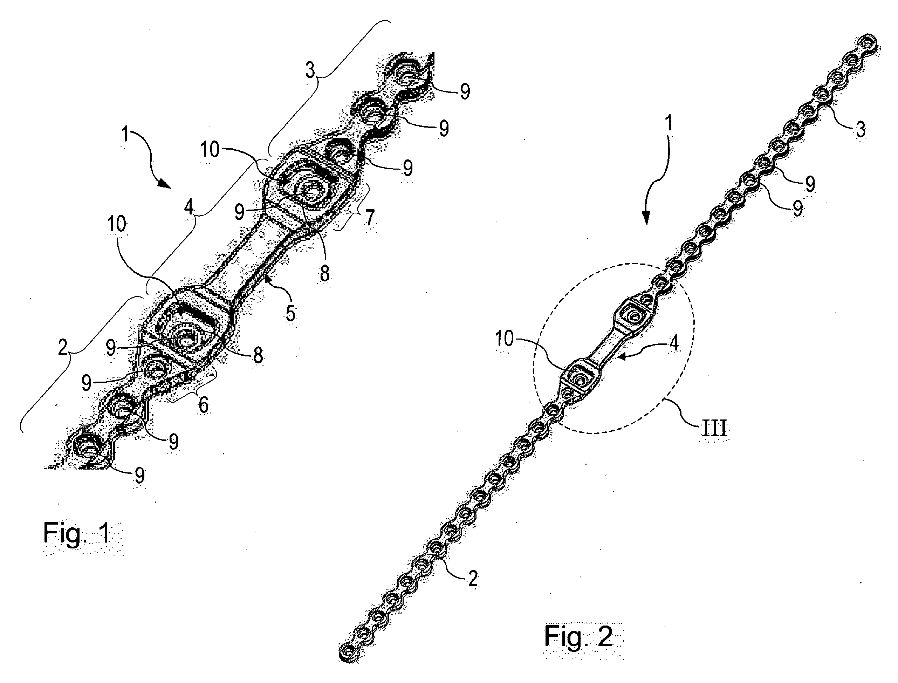

In Fig. 1 ist eine erste Ausführungsform eines erfindungsgemäßen Brustbeinverschlusses 1 dargestellt. Der Brustbeinverschluss 1 ist nur in einem mittleren Abschnitt dargestellt. Der Brustbeinverschluss 1 ist aus Kunststoff und/oder Metall gefertigt, insbesondere einer Metalllegierung, im vorliegenden Fall aus einer Titanlegierung. Insbesondere ist die Verwendung von hochtemperaturbeständigen thermoplastischen Kunststoffen, etwa Polyetheretherketon (PEEK) oder seinen Derivaten, alleine oder in Kombination mit anderen Werkstoffen denkbar. Auf die Biokompatibilät ist dabei zu achten.

Der Brustbeinverschluss ist ein einstückiges Bauteil, das einen ersten Befestigungsbereich 2 und einen zweiten Befestigungsbereich 3 aufweist. Die beiden Befestigungsbereiche 2 und 3 sind über ein Durchtrennungsstellenteil bzw. Entnahmeteil 4 miteinander verbunden. Das Durchtrennungsstellenteil 4 weist einen geschwächten Durchmesserbereich 5 auf. Dort ist der Querschnitt kleiner als im umgebenden Material.

Beide Befestigungsbereiche 2 und 3 weisen an deren aufeinander zu ausgerichteten Endbereichen 6 und 7 jeweils einen Verlängerungsabschnitt 8 auf.

Der Verlängerungsabschnitt 8 ist zumindest teilweise durch das Durchtrennungsstellenteil 4 bedeckt, wobei oberhalb des Verlängerungsabschnittes, eine Zugänglichkeit zu einem Loch 9 im Verlängerungsabschnitt gewährleistend, Ausnehmunggen 10, nach Art von Fenstern vorhanden sind. Während in jedem Verlängerungsabschnitt 8 ein Loch 9 vorhanden ist, sind weitere Löcher 9 auch in den restlichen Befestigungsbereichen 2 und 3 vorhanden. Die Löcher 9 sind zur Aufnahme von nicht dargestellten Knochenschrauben ausgebildet, wobei sie einen oberen größeren Durchmesserbereich und einen unteren kleineren Durchmesserbereich aufweisen, um einen Schraubenkopf der Knochenschrauben zurückzuhalten. Über die Knochenschrauben werden die beiden Befestigungsbereiche 2 und 3 an Rippen bzw. am Brustbein eines Patienten, also eines Menschen, befestigt. Die Knochenschraube kann über die fensterartige Ausnehmung 10 auch in das Loch 9 des jeweiligen Verlängerungsabschnitts 8 eingeführt werden.

Während des Befestigens, wird der erste Befestigungsbereich 2 auf der einen Seite eines Spalts im Brustbein des Patienten befestigt, wohingegen der zweite Befestigungsbereich 3 auf der anderen Seite des Spalts angebracht ist. Wie in Fig. 2 gut zu erkennen ist, sind die Löcher 9 äquidistant voneinander beabstandet. Der Brustbeinverschluss 1 hat eine längliche Ausgestaltung, wobei die beiden Befestigungsbereiche 2 und 3 in gewissen Maßen biegbar sind, um einer mehrdimensionalen Kurvenform zu folgen. Dies ist von Vorteil, um eine Anbringung an den Rippen effizient zu gewährleisten. Die einzelnen Abschnitte des Brustbeinverschlusses 1 sind somit gelenkartig an die Kontur der Knochen und Knorpelstücke des Patienten anpassbar.

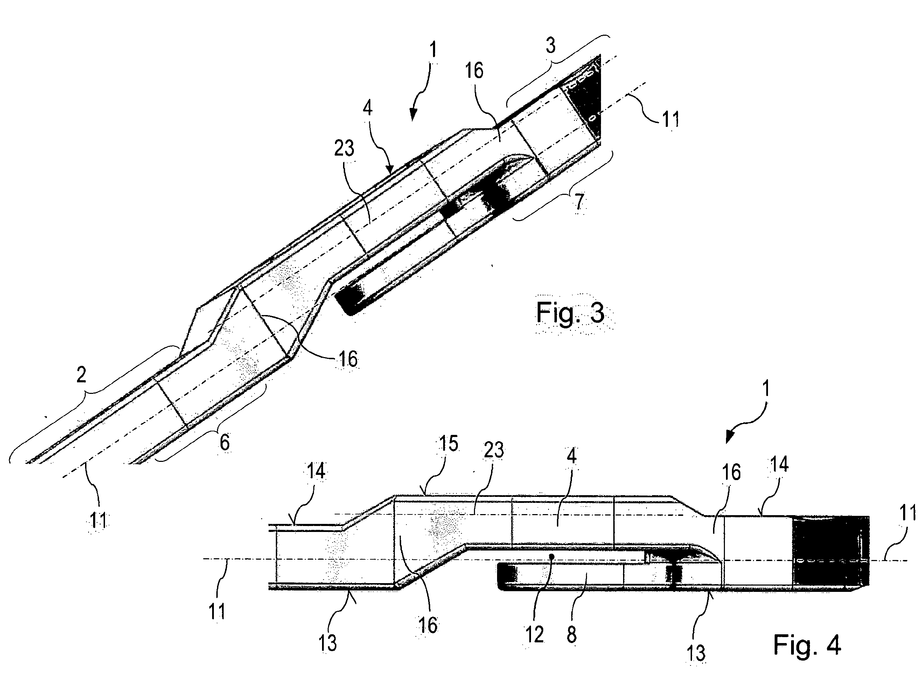

Dass der erste Befestigungsbereich 2 und der zweite Befestigungsbereich 3 in ein und derselben ersten Ebene 11, zumindest in der Gegend deren Endbereiche 6 und 7 befindlich ist, lässt sich auch den Fig. 3 und 4 entnehmen.

Zwischen dem Verlängerungsabschnitt 8 und dem Durchtrennungsstellenteil 4 ist eine Beabstandung 12, also ein Leerraum bzw. ein Spalt vorhanden. Während die Unterseite 13 der beiden Befestigungsbereiche 2 und 3 sowie des Verlängerungsabschnittes 8 auf derselben Höhe befindlich sind, ist oberhalb einer Oberseite 14 der beiden Befestigungsbereiche 2 und 3 die Oberfläche 15 des Durchtrennungsstellenteils 4 positioniert. Das Durchtrennungsstellenteil 4 geht über einen Knick 16 in die beiden Befestigungsbereiche 2 und 3 über.

Auch sei in Bezug auf die Fig. 3 und 4 noch einmal darauf hingewiesen, dass eine zweite Ebene 23 von einem Brustbein entfernt nach oben versetzt ist, wobei die zweite Ebene 23 durch das Durchtrennungsstellenteil 4 verläuft, wohingegen die erste Ebene 11 durch die beiden Befestigungsbereiche 2 und 3 verläuft.

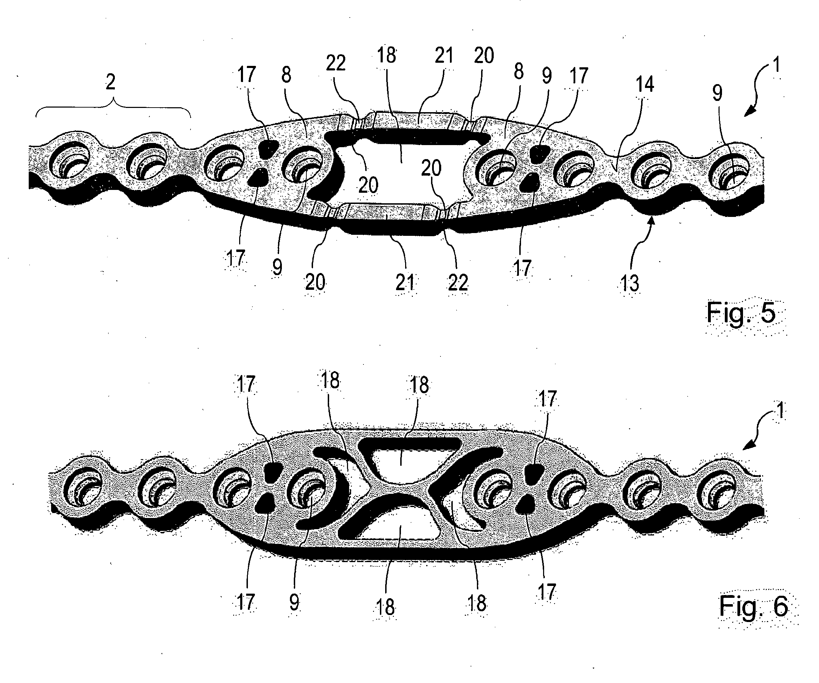

In den Fig. 5 bis 8 sind vier weitere Ausführungsbeispiele eines Brustbeinverschlusses dargestellt. In diesen Ausführungsformen ist zwischen dem Verlängerungsabschnitt 8 und den restlichen Befestigungsbereichen 2 und 3 eine Aussparung oder besser, wie vorhanden dargestellt, auf jeder Seite zwei Aussparungen 17 vorhanden, die sich komplett durch das Material des Brustbeinverschlusses von einer Oberseite 14 bis zur Unterseite 13 erstrecken.

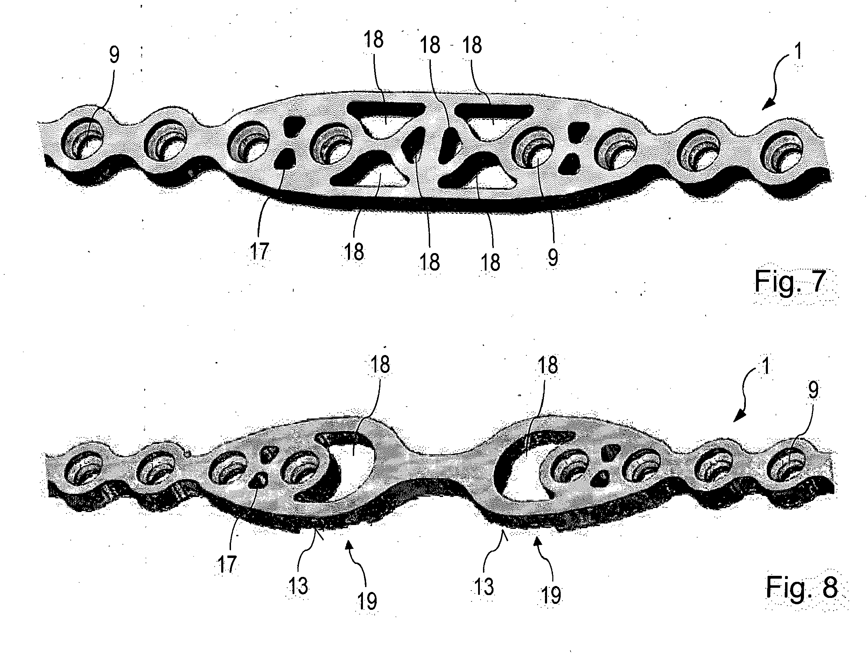

Im Durchtrennungsstellenteil 4 ist ein Hohlraum 18 vorhanden, der wie in den Ausführungsbeispielen der Fig. 6 und 8 dargestellt, auch mehrfach unterteilt sein kann. Wie insbesondere in den Ausführungsbeispielen der Fig. 6 und 7 erkenntlich, kann das Durchtrennungsstellenteil eine fachwerkartige Struktur aufweisen, oder wie insbesondere aus Fig. 8 hervorgeht, eine hantelartige oder doppel-gabelartige Struktur aufweisen. Die einzelnen Bestandteile der fachwerkartigen Struktur können dabei kurvig ausgebildet sein.

Eine doppel-gabelartige Durchtrennungsstellenteilarchitektur, wie in Fig. 8 dargestellt, kann auch an den Übergangsbereichen 19 zu den Befestigungsbereichen 2 und 3 Materialverknappungsbereiche aufweisen, also Bereiche reduzierter Dicke. Diese Bereiche reduzierter Dicke können auf einer Seite des Brustbeinverschlusses 1, auf beiden Seiten oder abwechselnd auf einer vorderen und einer hinteren Gabelseite vorhanden sein. Die Verdünnung ist dabei auf der Unterseite 13 ausgebildet.

Zurückkommend auf Fig. 5 sei noch angemerkt, dass der geschwächte Durchmesserbereich auch nach Art von Verdünnungsstellen 20 ausgeformt sein kann. Dabei verlaufen um einen entsprechenden Stab 21 herum Kerben 22.

### Bezugszeichenliste

- 1: Brustbeinverschluss
- 2: erster Befestigungsbereich
- 3: zweiter Befestigungsbereich
- 4: Durchtrennungsstellenteil
- 5: Durchmesserbereich
- 6: Endbereich des ersten Befestigungsbereiches
- 7: Endbereich des zweiten Befestigungsbereiches
- 8: Verlängerungsabschnitt
- 9: Loch
- 10: Ausnehmung
- 11: erste Ebene
- 12: Beabstandung
- 13: Unterseite
- 14: Oberseite
- 15: Oberfläche
- 16: Knick
- 17: Aussparung
- 18: Hohlraum
- 19: Übergangsbereich
- 20: Verdünnungsstelle
- 21: Stab
- 22: Kerbe
- 23: zweite Ebene

## Patentansprüche

1. Brustbeinverschluss (1) zum Verschließen eines Spaltes in einem Brustbein, wobei in einem ersten und in einem zweiten Befestigungsbereich (2, 3) zumindest je ein Loch (9) zum Aufnehmen von Knochenschrauben vorhanden ist, wobei die beiden Befestigungsbereiche (2, 3) auf je einer Seite des Spaltes anschraubbar sind, wobei ein die beiden Befestigungsbereiche (2, 3) verbindendes, integral mit diesen verbundenen Durchtrennungsstellenteil (4) vorhanden ist, **dadurch gekennzeichnet, dass** einer der beiden Befestigungsbereiche (2, 3) einen Verlängerungsabschnitt (8) aufweist, der zumindest teilweise durch das Durchtrennungsstellenteil (4) bedeckt ist, wobei der Verlängerungsabschnitt (8) vom Durchtrennungsstellenteil (4) beabstandet ist und am Brustbein in Anlage bringbar ist.

2. Brustbeinverschluss (1) nach Anspruch 1, **dadurch gekennzeichnet, dass** das Durchtrennungsstellenteil (4) einen geschwächten Durchmesserbereich (5) aufweist.

3. Brustbeinverschluss (1) nach einem der Ansprüche 1 oder 2, **dadurch gekennzeichnet, dass** die beiden Befestigungsbereiche (2, 3) zumindest, in den aufeinander zu zeigenden Endbereichen (6, 7) in einer gemeinsamen ersten Ebene (11) liegen.

4. Brustbeinverschluss (1) nach Anspruch 3, **dadurch gekennzeichnet, dass** das Durchtrennungsstellenteil (4) in einer zweiten Ebene (23) befindlich ist, die zur ersten Ebene (11) beabstandet und vorzugsweise parallel verlaufend ist.

5. Brustbeinverschluss (1) nach Anspruch 3 oder 4, **dadurch gekennzeichnet, dass** das Durchtrennungsstellenteil (4) auf der brustbeinfernen Seite der ersten Ebene (11) befindlich ist.

6. Brustbeinverschluss (1) nach einem der Ansprüche 1 bis 5, **dadurch gekennzeichnet, dass** im Verlängerungsabschnitt (8) ein Loch (9) für eine Knochenschraube vorhanden ist.

7. Brustbeinverschluss (1) nach einem der Ansprüche 1 bis 6, **dadurch gekennzeichnet, dass** in jedem der beiden länglichen, in Flucht zueinander ausgerichteten Befestigungsabschnitte (2, 3) eine Vielzahl gleichmäßig verteilter Löcher (9) vorhanden sind.

8. Brustbeinverschluss (1) nach einem der Ansprüche 1 bis 7, **dadurch gekennzeichnet, dass** das Durchtrennungsstellenteil (4) eine maximale Breite aufweist, die größer ist, als die Breite der Befestigungsbereiche (2, 3).

9. Brustbeinverschluss (1) nach einem der Ansprüche 1 bis 8, **dadurch gekennzeichnet, dass** das Durchtrennungsstellenteil (4) zumindest einen Hohlraum (18) oder eine Vielzahl an Hohlräumen (18) aufweist.

10. Brustbeinverschluss (1) nach einem der Ansprüche 1 bis 9, **dadurch gekennzeichnet, dass** das Durchtrennungsstellenteil (4) zwei Stäbe (21) aufweist, die vorzugsweise die maximale Breite des Durchtrennungsstellenteils (4) festlegen.

11. Brustbeinverschluss (1) nach Anspruch 10, **dadurch gekennzeichnet, dass** ein Stab (21) oder jeder Stab (21) über eine oder zwei Verdünnungsstellen (20) mit einem Befestigungsbereich (2, 3) oder beiden Befestigungsbereichen (2, 3) verbunden sind.

12. Brustbeinverschluss (1) nach einem der Ansprüche 1 bis 11, **dadurch gekennzeichnet, dass** das Durchtrennungsstellenbauteil (4) eine fachwerkartige Struktur oder eine hantelartige oder doppel-gabelartige Struktur aufweist.

13. Brustbeinverschluss (1) nach einem der Ansprüche 10 bis 12, **dadurch gekennzeichnet, dass** die Durchtrennungsstelle und/oder die Verdünnungsstelle (20) in einem außermittigen Bereich des Durchtrennungsstellenteils (4) verbunden ist.

14. Brustbeinverschluss (1) nach einem der Ansprüche 1 bis 13, **dadurch gekennzeichnet, dass** der Brustbeinverschluss aus einer Metalllegierung, wie einer Titanlegierung, ausgebildet ist.

## Claims

1. A sternal closure (1) for closing a cleft in a sternum, wherein in each of a first and a second securing area (2, 3) at least one hole (9) for receiving bone screws is provided, the two securing areas (2, 3) being adapted to be screwed on a respective side of the cleft, wherein a severing site member (4) joining the two securing areas (2, 3) and being integrally joined to the latter is provided, **characterized in that** either of the two securing areas (2, 3) includes an extension portion (8) which is at least partially covered by the severing site member (4), wherein the extension portion (8) is spaced apart from the severing site member (4) and can be made to contact the sternum.

2. The sternal closure (1) according to claim 1, **characterized in that** the severing site member (4) includes a weakened diametric region (5).

3. The sternal closure (1) according to one of the claims 1 or 2, **characterized in that** the two securing areas (2, 3) are located in a joint first plane (11) at least in the end zones (6, 7) facing each other.

4. The sternal closure (1) according to claim 3, **characterized in that** the severing site member (4) is provided in a second plane (23) which is spaced from the first plane (11) and preferably extends in parallel thereto.

5. The sternal closure (1) according to claim 3 or 4, **characterized in that** the severing site member (4) is provided on the side of the first plane (11) distant from the sternum.

6. The sternal closure (1) according to one of the claims 1 to 5, **characterized in that** in the extension portion (8) a hole (9) for a bone screw is provided.

7. The sternal closure (1) according to one of the claims 1 to 6, **characterized in that** in each of the two oblong securing portions (2, 3) aligned to each other a plurality of evenly distributed holes (9) is provided.

8. The sternal closure (1) according to one of the claims 1 to 7, **characterized in that** the severing site member (4) has a maximum width which is larger than the width of the securing areas (2, 3).

9. The sternal closure (1) according to one of the claims 1 to 8, **characterized in that** the severing site member (4) includes at least one cavity (18) or a plurality of cavities (18).

10. The sternal closure (1) according to one of the claims 1 to 9, **characterized in that** the severing site member (4) includes two rods (21) preferably defining the maximum width of the severing site member (4).

11. The sternal closure (1) according to claim 10, **characterized in that** one rod (21) or each rod (21) is connected to one securing area (2, 3) or both securing areas (2, 3) via one or two thinning sites (20).

12. The sternal closure (1) according to one of the claims 1 to 11, **characterized in that** the severing site member (4) has a trussed structure or a dumb-bell shaped or double fork-like structure.

13. The sternal closure (1) according to one of the claims 11 to 12, **characterized in that** the severing site and/or the thinning site (20) is provided in an off-center area of the severing site member (4).

14. The sternal closure (1) according to one of the claims 1 to 13, **characterized in that** the sternal closure is made of a metal alloy such as a titanium alloy.

## Revendications

1. Fermeture pour sternum (1) permettant de fermer une fente dans un sternum, au moins un trou (9), destiné à recevoir des vis à os, étant disponible respectivement dans une première et une deuxième zone de fixation (2, 3), les deux zones de fixation (2, 3) pouvant être vissées chacune sur un côté de la fente, une pièce à zone de sectionnement (4), reliant les deux zones de fixation (2, 3) et reliée intégralement à celles-ci, étant disponible, **caractérisée en ce que** l'une des deux zones de fixation (2, 3) comporte une partie de prolongement (8) masquée au moins partiellement par la pièce à zone de sectionnement (4), ladite partie de prolongement (8) étant écartée de la pièce à zone de sectionnement (4) et pouvant être amenée en appui sur le sternum.

2. Fermeture pour sternum (1) selon la revendication 1, **caractérisée en ce que** la pièce à zone de sectionnement (4) comporte une zone de diamètre (5) affaiblie.

3. Fermeture pour sternum (1) selon la revendication 1 ou 2, **caractérisée en ce que** les deux zones de fixation (2, 3), au moins dans les zones d'extrémité (6, 7) orientées l'une vers l'autre, sont situées dans un premier plan (11) commun.

4. Fermeture pour sternum (1) selon la revendication 3, **caractérisée en ce que** la pièce à zone de sectionnement (4) est située dans un deuxième plan (23) qui est distant du premier plan (11) et est de préférence parallèle à celui-ci.

5. Fermeture pour sternum (1) selon la revendication 3 ou 4, **caractérisée en ce que** la pièce à zone de sectionnement (4) est située dans le premier plan (11) sur le côté éloigné du sternum.

6. Fermeture pour sternum (1) selon l'une quelconque des revendications 1 à 5, **caractérisée en ce qu'**un trou (9) pour une vis à os est disponible dans la partie de prolongement (8).

7. Fermeture pour sternum (1) selon l'une quelconque des revendications 1 à 6, **caractérisée en ce qu'**une pluralité de trous (9), répartis régulièrement, sont disponibles dans chacune des deux zones de fixation (2, 3) allongées alignées l'une avec l'autre.

8. Fermeture pour sternum (1) selon l'une quelconque des revendications 1 à 7, **caractérisée en ce que** la pièce à zone de sectionnement (4) présente une largeur maximale, qui est supérieure à la largeur des zones de fixation (2, 3).

9. Fermeture pour sternum (1) selon l'une quelconque des revendications 1 à 8, **caractérisée en ce que** la pièce à zone de sectionnement (4) comporte au moins une cavité (18) ou une pluralité de cavités (18).

10. Fermeture pour sternum (1) selon l'une quelconque des revendications 1 à 9, **caractérisée en ce que** la pièce à zone de sectionnement (4) comporte deux barrettes (21) qui définissent de préférence la largeur maximale de la pièce à zone de sectionnement (4).

11. Fermeture pour sternum (1) selon la revendication 10, **caractérisée en ce qu'**une barrette (21) ou chaque barrette (21) est reliée, par l'intermédiaire d'une ou de deux zones à épaisseur réduite (20), à une zone de fixation (2, 3) ou aux deux zones de fixation (2, 3).

12. Fermeture pour sternum (1) selon l'une quelconque des revendications 1 à 11, **caractérisée en ce que** la pièce à zone de sectionnement (4) comporte une structure en treillis ou une structure en forme d'haltère ou de double fourche.

13. Fermeture pour sternum (1) selon l'une quelconque des revendications 10 à 12, **caractérisée en ce que** la zone de sectionnement et/ou la zone à épaisseur réduite (20) est reliée dans une zone excentrée de la pièce à zone de sectionnement (4).

14. Fermeture pour sternum (1) selon l'une quelconque des revendications 1 à 13, **caractérisée en ce que** la fermeture pour sternum est réalisée dans un alliage métallique, tel qu'un alliage de titane.
